# EUROPEAN PATENT APPLICATION

(11) **EP 1 216 989 A1**
(43) Date of publication of application: **26.06.2002**
(21) Application number: 01310598.6
(22) Date of filing: 19.12.2001
(51) Int. Cl.: C07C 381/12, G01N 30/00

(54) **Buffer compositions**

(30) Priority: 20.12.2000 US 257006 P
(71) Applicant: THE TEXAS A & M UNIVERSITY SYSTEMS, College Station, TX 77843 (US)
(72) Inventor: Vigh, Gyula, Magnolia, Texas 77355 (US)
(74) Representative: W.P. Thompson & Co.

(57) **Abstract**

New non-interfering buffer and buffer systems having volatile combustion products are disclosed for use in analytical techniques utilizing element specific detectors. The new buffers and buffer systems are free of heteroatoms except for O or O and S and are free of metal salt containing counterions. Also, analytical techniques are disclosed using the new buffers and buffer systems.

## Description

### BACKGROUND OF THE INVENTION

### 1. Field of the Invention

The present invention relates to buffers and families of buffers having volatile combustion products that are metal-free and heteroatom-free, except O or O and S, to be used in conjunction with gas-phase or vapor-phase element-specific detectors.

More particularly, the present invention relates to buffers and families of buffers having volatile combustion products that are metal-free and heteroatom-free, except O or O and S, to be used in conjunction with gas-phase or vapor-phase element-specific detectors (ESDs), such as nitrogen-selective gas-phase chemiluminescence detectors, sulfur-selective gas-phase chemiluminescence detectors, nitrogen-phosphorus thermoionic detectors, electron-capture detectors, atomic cmission plasma detectors, inductively-coupled plasma-mass spectrometric (ICP-MS) detectors, *etc.,* where the buffers include nitrogen free containing weak acids and nitrogen free and metal atom free counterions having volatile combustion products.

### 2. Description of the Related Art

In order to form a buffer system, a weak acid and its conjugate base or a weak base and its conjugate weak acid must be present. The counterion of the conjugate weak acid (and the counterion of the conjugate weak base) can be derived either from strong electrolytes or weak electrolytes. The typical buffer systems utilize various weak acids (with pKₐ values in the 1 to 13 range) or weak bases (with pK_{b} values in the 1 to 13 range).

Typically, the weak acids are inorganic weak acids (such as phosphoric acid, carbonic acid or boric acid), carboxylic acids or phenolics. The counterions of their conjugate bases arc typically alkali metal cations or alkaline earth metal cations, protonated amines or quatemary ammonium cations.

Typically, the weak bases are amines and the counterions of their conjugate acids are typically hydroxyl ions, carbonate ions, hydrogen carbonate ions, hydrogen sulfate ions, sulfate ions, sulfonate ions, halide ions, etc.

The use of amines or quaternary ammonium compounds with nitrogen-selective detectors is undesirable, because they contribute a high background signal level. Alkali metal cations or alkaline earth metal cations should not be used with gas-phase ESDs, because the combustion products of these metals are not sufficiently volatile to leave the gas-phase detector systems as vapors or gases and they contaminate the detector systems.

Thus, there is a need in the art for buffers and buffer systems where the buffer components are all combustible, the combustion products are all volatile and do not significantly interfere with elements specific detection.

### SUMMARY OF THE INVENTION

The present invention provide a composition comprising a strong electrolytic cationic salt of a weak acid, where the composition is metal-free and free of heteroatoms, except O or O and S and has volatile combustion products.

The present invention provides a family of novel buffet compositions having volatile combustion products which are free of heteroatoms, except O or O and S, to be used in conjunction with gas-phase or vapor-phase, element-specific detectors (ESDs), such as nitrogen-selective gas-phase chemiluminescence detectors, sulfur-selective gas-phase chemiluminescence detectors, nitrogen-phosphorus thermoionic detectors, electron-capture detectors, atomic emission plasma detectors, inductively-coupled plasma-mass spectrometric (ICP-MS) detectors, *etc*.

The present invention also provides analytical applications using the new buffers including chromatographic, electrophoretic and/or extractive separation or flow-injection analysis of heteroatom-containing (other than O or O and S) analytes and/or their quantitative determination by coupled gas-phase or vapor-phase ESDs.

The present invention also provides new buffer compositions having volatile combustion products including an acid having volatile combustion products and having only O or O and S heteroatoms, and a counterion having volatile combustion products and having only O or O and S heteroatoms.

The present invention also provides a buffer system including two or more buffer compositions of this invention.

The present invention also provides new buffer compositions having volatile combustion products including an acid having volatile combustion products selected from the group consisting of carboxylic acids, phenols, half esters of sulfuric acid, and acidic hydroxy compounds, and a counterion having volatile combustion products selected from the group consisting of oxonium ions, sulfonium ions and sulfoxonium ions.

The present invention also provides a buffer system including two or more acids having volatile combustion products selected from the group consisting of carboxylic acids, phenols, half esters of sulfuric acid, and acidic hydroxy compounds, and one or more counterions having volatile combustion products selected from the group consisting of oxonium ions, sulfonium ions and sulfoxonium ions, designed to cover a given range of pH values.

The present invention also provides a buffer system including two or more acids having volatile combustion products selected from the group consisting of carboxylic acids, phenols, half esters of sulfuric acid, and acidic hydroxy compounds, and one or more counterions having volatile combustion products selected from the group consisting of oxonium ions, sulfonium ions and sulfoxonium ions, designed to cover pH values between about 1 to about 13.

The present invention also provides an analytical system for detecting an analyte including a combustion zone where an analyte, together with a buffer or buffer system of this invention is converted to their corresponding volatile combustion products and a detector capable of detecting at least one of the corresponding volatile combustion products.

The present invention also provides a method including the steps of producing a solution including a sample and a buffer or buffer system of this invention, combusting the sample to its corresponding volatile combustion products and detecting one or more volatile combustion products of at least one analyte in the sample.

The present invention also provides a method including the steps of producing a solution including a sample and a buffer or buffer system of this invention, combusting the sample and the buffer or buffer system to their corresponding volatile combustion products, converting one or more volatile combustion products of the sample into a transformate and detecting one or more transformate.

The present invention also relates to an analytical system for detecting an analyte including a separation apparatus where a sample, together with a buffer or buffer system of this invention, is separated into its constituents, a combustion zone where each constituent is converted to its corresponding volatile combustion products and a detector capable of detecting at least one of the corresponding volatile combustion products of at least one constituent or analyte of the sample.

The present invention also relates to an analytical system for detecting an analyte including a separation apparatus where an analyte, together with a buffer or buffer system of this invention is separated into its constituents, a combustion zone where each constituent is converted to its corresponding volatile combustion products, a transformation zone where one or more of the corresponding volatile combustion products of the analyte are converted into a transformate and a detector capable of detecting at least one of the transformates.

### DESCRIPTION OF THE DRAWINGS

The invention can be better understood with reference to the following detailed description together with the appended illustrative drawings in which like elements are numbered the same:
Figure 1 graphically depicts the 300 MHz ¹H NMR spectrum of TMSOH, dissolved in D₂O;
Figure 2 graphically depicts the ¹H and ¹³C NMR spectra of the trimethylsulfonium salt of diethylmalonic acid;
Figure 3 graphically depicts the ¹H and ¹³C NMR spectra of the trimethylsulfonium salt of 1,2,3,4-butane tetracarboxylic acid;
Figure 4 graphically depicts the ¹H and ¹³C NMR spectra of the trimethylsulfonium salt of cis-1,2,3,4,5,6-cyclohexyl hexacarboxylic acid; and
Figure 5 graphically depicts the buffer capacity vs. pH curves for diethylmalonic acid, 1,2,3,4-butane tetracarboxylic acid, and cis-1,2,3,4,5,6-cyclohexyl hexacarboxylic acid.

### DETAILED DESCRIPTION OF THE INVENTION

The inventor has found that a new class of buffers and buffer systems can be constructed that allow element specific detection of element constituents without the typical contamination of the analyte by buffer components containing the element to be detected or containing components or their combustion products that lack the needed volatility to pass through the analytical detection system without fouling it. The heteroatom-free (except for O or O and S) buffer compositions having volatile combustion products make possible the use of element specific detection (ESDs) in areas which hitherto have been unaccessible to ESDs, because the buffer compositions included heteroatom-containing compounds (*e.g*., nitrogen compounds, such as amines and quaternary ammonium compounds), or metal-containing compounds having non-volatile combustion products. In addition to producing a high background signal in the ESDS, the heteroatom- or metal-containing compounds often yield combustion products that cannot leave the ESDs as gases or vapors and contaminate the ESDs.

The present invention broadly relates to compounds for forming buffers and buffer systems of this invention, where the compounds comprise salts of weak acid which are metal-free and heteroatom-free (except for O or O and S) and generate volatile combustion products when combusted or oxidized.

The present invention also broadly relates to buffers and families of buffers including components having volatile combustion products that are metal-free and heteroatom-free, except O or O and S, to be used in conjunction with gas-phase or vapor-phase element-specific detectors.

The present invention broadly relates to buffers and families of buffers including components having volatile combustion products that are metal-free and heteroatom-free, except O or O and S, to be used in conjunction with gas-phase or vapor-phase element-specific detectors.

The present invention also relates to a composition comprising a cation salt of a weak acid, where the salt is metal-atom-free and free of heteroatoms, except O or O and S and has volatile combustion products, where the cation salt and the weak acid are as described herein.

The present invention also relates to a buffer composition comprising a compound that is, to be used in conjunction with gas-phase or vapor-phase, element-specific detectors (ESDs), such as nitrogen-selective gas-phase chemiluminescence detectors, sulfur-selective gas-phase chemiluminescence detectors, nitrogen-phosphorus thermoionic detectors, electron-capture detectors, atomic emission plasma detectors, inductively-coupled plasma -mass spectrometric (ICP-MS) detectors, or the like.

The present invention also relates to buffer systems including two or more buffers of this invention.

The present invention also provides an analytical system for detecting an analyte including a combustion chamber where an analyte, together with a buffer or buffer system of this invention is converted to their corresponding volatile combustion products and a detector capable of detecting at least one of the corresponding volatile combustion products of the analyte.

The present invention also provides a method including the steps of combusting a composition including an analyte and a buffer or buffer system of this invention to produce volatile combustion products and detecting one or more elements in the volatile combustion products of the analyte.

The present invention also relates to an analytical system for detecting an analyte including a separation apparatus where a sample, together with a buffer or buffer system of this invention is separated into its constituents, a combustion chamber where each constituent is converted to its corresponding volatile combustion products and a detector capable of detecting at least one of the corresponding volatile combustion products of the analyte.

The present invention also relates to an analytical application using the buffers of this invention comprising chromatographic, electrophoretic and/or extractive separation or flow-injection analysis of heteroatom-containing (other than O or O and S) analytes and/or their quantitative determination by coupled gas-phase or vapor-phase ESDs.

The present invention also relates to an analytical system for detecting an analyte including a separation apparatus where a sample together with a buffer or buffer system of this invention is separated into its constituents, a combustion zone where each constituent is converted to its corresponding volatile combustion product(s), a transformation zone where one or more of the corresponding volatile combustion product(s) of the analyte are converted into a transformate and a detector capable of detecting at least one of the transformates. Such transformation zone can include reduction zone, where one or more combustion product is reduced to a reduced product capable of being detected by a element-specific-detector. Reduction is especially useful in systems containing sulfur or phosphorus, Additional details on combustion zone, transformation zone and detector systems can be found in U.S. Pat. Nos. 4,352,779, 4,678,756, 4,950,456, 4,91,077. 4,904,606, 4,914,037, 5,227,135. 5,310,683, 5,330,714, 5,424,217, and 5,916,523, incorporated herein by reference.

The term metal-atom-free or free of metal atoms means that the buffers are free of metal atoms and their corresponding ions. Conventional buffers typically include metal ions and not the "atom" *per se*. However, the term metal atom free In the present invention includes metal atom and/or their corresponding ionized forms or metal ions.

The buffers of this invention arc characterized by at least the following properties: (1) each buffer component is combustible; (2) each combustion product of each buffer component is volatile (e.g., CO₂, H₂O, SₓO_{y}) at the operating conditions of the detection system; (3) each volatile combustion product is free of heteroatoms, except O or O and S, or metal atoms or ions; (4) each buffer provides adequate buffering over its designed pH range; (5) a combination of buffers can be constructed to provide adequate buffering over the analytically useful pH range from about 1 to about 13; and (6) each buffer and each combination of buffers permits the variation of the hydrophobicity and/or electrophoretic characteristics of the counterion(s) to match the requirements or constraints of the separation system used.

In order to form a buffer system, a weak acid and its conjugate base or a weak base and its conjugate acid must be present. The counterion of the conjugate weak acid (and the counterion of the conjugate base) can be derived either from strong electrolytes or weak electrolytes. The typical buffer systems utilize various weak acids (with pKₐ values in the about 1 to about 13 range) or weak bases (with pK_{b} values in the about 1 to about 13 range).

Typically, the weak acids are inorganic Weak acids (such as phosphoric acid, carbonic acid or boric acid), carboxylic acids or phenolies. The counterions of their conjugate bases are typically alkali metal cations or alkaline earth metal cations, protonated amines or quaternary ammonium cations. The weak bases are typically amines and the counterions of their conjugate acids are typically hydroxyl ions, carbonate ions, hydrogen carbonate ions, sulfate ions, hydrogen sulfate ions, sulfonate ions, halide ions, or the like.

The use of amines or quaternary ammonium compounds with nitrogen-selective detectors is undesirable, because they contribute a, high background signal level. Alkali or alkaline earth metal cations should not be used with gas-phase ESDs, because the combustion products of these metals are not sufficiently volatile to leave the gas-phase detector systems as vapors or gases and they tend to contaminate system components including the detector systems.

Although the buffers of the present invention can be tailored to any given pH value or range, it is preferable that the weak acids selected for the heteroatom-free (except for O, S or O and S) buffers having volatile combustion products of this invention have pKₐ values that are equidistantly spaced approximately 1 unit apart. When they are paired with their respective heteroatom-free (except for O, S or O and S) permanent cation salts having volatile combustion products, buffers can be created which offer adequate buffering capacities over the analytically most useful pH range between about 1 and about 13.

Suitable weak acids for use in the construction of buffers of this invention include, without limitation, carboxylic acids containing only carbon, hydrogen and oxygen or carbon, hydrogen, oxygen and sulfur atoms, and acidic hydroxy compounds containing only carbon, hydrogen and oxygen or carbon, hydrogen, oxygen and sulfur atoms. The carboxylic acids include, without limitation, mono-, oligo-, or polycarboxy alkanes, alkenes, or alkyncs, mono-, oligo- or polycarboxy cycloalkanes, cycloalkenes, cycloalkynes, mono-, oligo-, or polycarboxy aromatics, Heteroatom-containing analogs thereof, where the heteroatoms are O and/or S, or mixtures or combinations thereof.

Preferred weak acids for use in the construction of buffers of this invention include, without limitation, diethylmalonic acid having pKa₁ = 2.21 and pKa₂ = 7.29; 1,2,3,4-butane tetracarboxylic acid having pKa₁ = 3.16, pKa₂ = 4.11, pKa₃ = 5.16, pKa₄ = 6.29; 3-hydroxy-2-methyl-4-pyrone (maltol) having pKa₁ = 8.4; 4-hydroxy benzoic acid having pKa₁ = 4.53, pKa₂ =9.31; carbonic acid having pKa₁ = 6.1, pKa₂ =9.9; and cis-cyclohexyl hexacarboxylic acid having pKa values in a range between about 1 and about 13.

Suitable strong bases having volatile combustion products to create the cation salts of the selected weak acids to be used in this invention include, without limitation, any relatively stable carbenium hydroxide, any relatively stable oxonium hydroxide, any relatively stable sulfonium hydroxide and any telatively stable sulfoxonium hydroxide. The term relatively stable means that no more than about 15 wt.% of the salt decomposes during an analysis under the analytical conditions used, preferably, no more than 10 wt,% of the salt decomposes during analysis, and particularly, no more than 5 wt.%of the salt decomposes during analysis.

Preferred strong bases having volatile combustion products to be used to create the cation salts of the selected weak acids for use in this invention include, without limitation, pyrillium hydroxides, sulfonium hydroxides, and sulfoxonium hydroxides.

Suitable suflonium hydroxides include, without limitation, R,R',R"-sulfonium hydroxides, where R,R' and R" are the same or different alkyl group having from 1 to about 30 carbon atoms, aryl group having from 6 to about 30 carbon atoms, aralkyl group having from 7 to about 30 carbon atoms, alkaryl group having from 7 to about 30 carbon atoms, a polyalkylene glycol group, a polyalkyleneoxide group, or mixtures or combinations thereof.

Suitable sufloxonium hydroxides include, without limitation, R,R',R"-sulfoxonium hydroxides, where R, R' and R" are the same or different alkyl group having from 1 to about 30 carbon atoms, aryl group having from 6 to about 30 carbon atoms, aralkyl group having from 7 to about 30 carbon atoms, alkaryl group having from 7 to about 30 carbon atoms, a polyalkylene glycol group, a polyalkylencoxide group, or mixtures or combinations thereof.

Exemplary examples of strong bases include, without limitation, 2,4,6-triphenylpytillium hydroxide, which can be prepared from 2,4,6-triphenylpyrillium tetrafluoroborate by ion exchange on a hydroxide-form strong anion exchanger column; trialkylsulfonium hydroxide, where the alkyl groups are the same or different and have from 1 to about 30 carbon atoms, such as trimethylsulfonium hydroxide, which can be prepared from trimethylsulfonium iodide by ion exchange on a hydroxide-form strong anion exchanger column, diethylmethylsulfonium hydroxide, dipropylmethylsulfonium hydroxide, dibutylmethylsulfonium hydroxide, which can be prepared from their respective alkyl sulfide and methyl iodide and ion exchanged on a hydroxide-form strong anion exchanger column; trialkylsulfoxonium hydroxides, where the alkyl groups are the same or different and have from 1 to about 30 carbon atoms, such as trimethylsulfoxonium hydroxide, which can be prepared from trimethylsulfoxonium iodide by ion exchange on a hydroxide-form strong anion exchanger column, diethylmethylsulfoxonium hydroxide, dipropylmethylsulfoxonium hydroxide, dibutylmethylsulfoxonium hydroxide, which can be prepared from their respective alkyl sulfoxide and methyl iodide and ion exchanged on a hydroxide-form strong anion exchanger column; (alkoxycarbonylmethyl) dialkylsulfonium hydroxides, where the alkyl groups are the same or different and have from 1 to about 30 carbon atoms and the alkoxy group have between 1 and 30 carbon atoms, such as ethoxycarbonylmethyl) dimethylsulfonium hydroxide, which can be prepared from (ethoxycarbonylmethyl) dimethylsulfonium bromide by ion exchange on a hydroxide-form strong anion exchanger column; any of the above compounds, where one or more of the alkyl groups is replaced by a polyalkylene glycol such as polyethylene glycol, polypropylene glycol or the like or a polyalkyleneoxide chain such as a polyethyleneoxide chain, a polypropyleneoxide chain, a polybutyleneoxide chain or the like to adjust the hydrophobic-hydrophilic balance and/or electrophoretic characteristics or properties of the cation(s) to bring about improved solubility and/or improved electrophoretic performance.

The buffers can be produced in a number of ways, the following two ways being preferred exemplary methods for making the buffers of this invention:
A. Preparation from the respective weak acids and the strong base(s):
   1. Select a weak acid of this invention according to the pH value desired;
   2. Select the desired buffer concentration;
   3. Dissolve the weak acid in water, or in the desired hydroorganic solvent mixture or in the desired organic solvent; and
   4. Titrate the solution to the desired pH with (a) selected strong base(s) of this invention.
B. Preparation from the respective weak acids and their cation salts:

1. Select a weak acid of this invention according to the pH value desired;
2. Select the desired buffer concentration;
3. Calculate the amount of weak acid and its salt required to reach the desired pH using a secondary chemical equillbrium approach; and
4. Dissolve the calculated amount of weak acid and its salts in water, or in the desired hydroorganic solvent mixture or in the desired organic solvent.

By using these heteroatom-free (except for O and/or S) buffers having volatile combustion products, heteroatom-containing analytes, such as nitrogen-containing analytes can be detected and quantified selectively even if they are not completely separated from other heteroatom-free compounds.

### EXPERIMENTAL SECTION

### Example 1

This example illustrates the preparation of a standardized trimethylsulfonium hydroxide (TMSOH) solution.

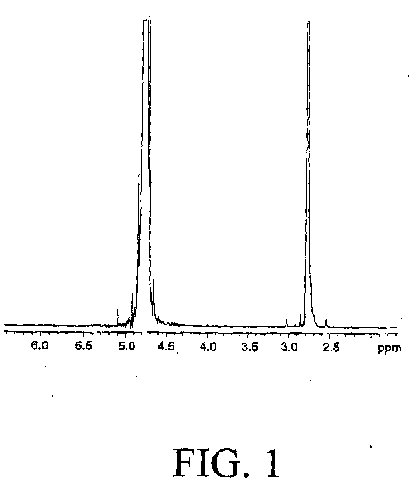

61.221 g of trimethylsulfonium iodode (TMSI) was dissolved in 250 mL of deionized water. The solution was percolated through a 4 cm intemal diameter glass column, packed with 1150 mL of 20-40 mesh Dowex 1 ion exchange resin in the hydroxide form, at a flow rate of 3,3 mL/min. The effluent was collected and the column was rinsed with deionized water until the pH of the effluent dropped below pH 7. The effluents were combined and concentrated, at room temperature, in a rotary evaporator to an approximate volume of 350 mL. The concentrated TMSOH solution was standardized by titrating a 10 mL aliquot of 0.14 M potassium hydrogen phthalate solution and recording the pH by a combination glass electrode and pH meter. The concentration of the concentrated TMSOH solution was found to be typically in the 1.3 to 1.5 M range. The 300 MHz ¹H and ¹³C NMR spectra of TMSOH, dissolved in D₂O, is shown in Figure 1.

### Example 2

This example illustrates the preparation of the trimethylsulfonium salt of diethylmalonic acid (DEMA(TMS)₂).

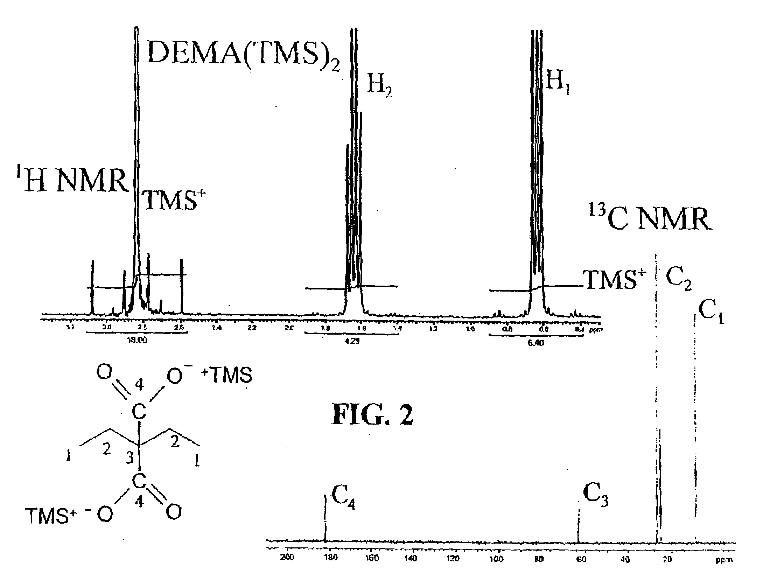

To 149,7 mL of a fresh standardized 1.277 M TMSOH solution prepared according to Example 1, was added 15 g of diethylmalonic acid (H₂DEMA). Ths mixture was stirred until a clear solution was obtained. The clear solution was lyophilized overnight to obtain solid DEMA(TMS)₂. The 300 MHz ¹H and ¹³C NMR spectra of DEMA(TMS)₂, dissolved in D₂O, are shown in Figure 2.

### Example 3

This example illustrates the preparation of the trimethylsulfonium salt of 1,2,3,4-butanetetracarboxylic acid (BTCA(TMS)₄).

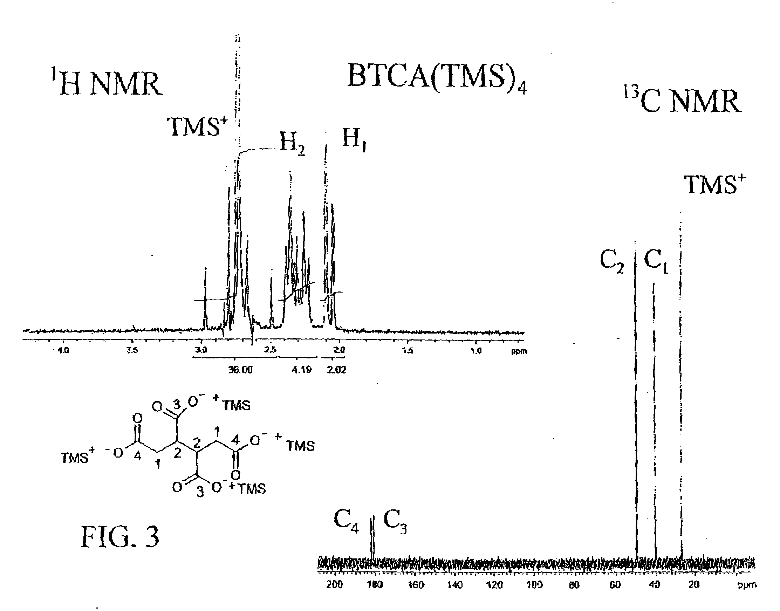

To 202,7 mL of a fresh standardized 1.277 M TMSOH solution prepared according to Example 1, was added 15 g of 1,2,3,4-butanetetracarboxylic acid (H₄BTCA). The mixture was stirred until a clear solution was obtained. The clear solution was lyophilized overnight to obtain solid BTCA(TMS)₄. The 300 MHz ¹H and ¹³C NMR spectra of BTCA(TMS)₄, dissolved in D₂O, are shown in Figure 3.

### Example 4

This example illustrates the preparation of the trimethylsulfonium salt of 1,2,3,4,5,6-cis-cyclohexylhexacarboxylic acid (CHHCA(TMS)₆).

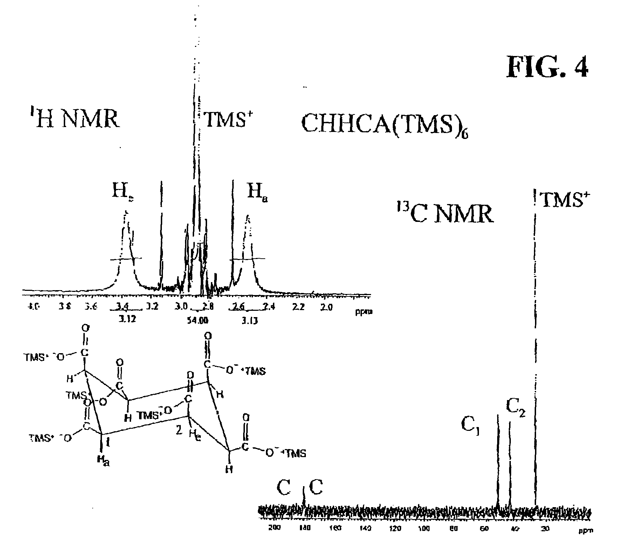

To 202.4 ml of a fresh standardized 1.277 M TMSOH solution prepared according to Example 1, was added 15,785 g of 1,2,3,4,5,6-cis-cyclohexylhexacarboxylic acid (H₆CHHCA⁺H₂O). The solution was stirred until a clear solution was obtained. The clear solution was lyophilized overnight to obtain solid CHHCA(TMS)₆. The 300 MHz ¹H and ¹³C NMR spectra of CHHCA(TMS)₆, dissolved in D₂O, are shown in Figure 4.

### Example 5

This example illustrates the preparation of a nominal pH = 2,21, H₂DEMA/HDEMA⁻¹ buffer based on diethylmalonic acid (H₂DEMA) and the trimethylsulfonium salt of diethylmalonic acid (DEMA(TMS)₂).

0.385 g of H₂DEMA (corresponding to 2.4 mmol) and 0.375 g of DEMA(TMS)₂ prepared according to Example 2 (corresponding to 1.2 mmol) were weighed out and added to a 100 mL volumetric flask. 90 mL of de-ionized water was added to the flask to dissolve the components. The flask was filled to the mark with de-ionized water. The pH of the solution was measured with a combination glass electrode and pH meter and found to be 2,19.

### Example 6

This example illustrates the preparation of a nominal pH = 6.54, HBTCA³⁻/BTCA⁴⁻ buffer based on 1,2,3,4-butanetetracarboxylic acid (H₄BTCA) and the trimethylsulfonium salt of 1,2,3,4-butanetetracarboxylic acid (BTCA(TMS)ₐ).

0.089 g of H₄BTCA and 1.410 g of BTCA(TMS)₄ prepared according to Example 3 was weighed out and added to a 100 mL volumetric flask. 90 mL deionized water was added to the flask to dissolve the components. The flask was filled to the mark wilh de-ionized water. The pH of the solution was measured with a combination glass electrode and pH meter and found to be 6.5.

### Example 7

This example illustrates the preparation of a nominal pH = 11.93, HCHHCA⁵⁻/CHHCA⁶⁻buffer based on 1,2,3,4,5,6-cis-cyclohexylhexacarboxylic acid(H₆CHHCA) and the trimethylsulfonium salt of 1,2,3,4,5,6-cis-cyclohexylhexacarboxylic acid (CHHCA(TMS)₆).

0.330 g of H₆CHHCA and 8.258 g of CHHCA(TMS)₆ prepared according to Example 4 was weighed out and added to a 100 mL volumetric flask. 90 mL deionized water was added to the flask to dissolve the components. The flask was filled to the mark with de-ionized water. The pH of the solution was measured with a combination glass electrode and pH meter and found to be 12.0.

### Example 8

This example illustrates the determination of the buffer capacity curves of the diethylmalonic acid, 1,2,3,4-butanetetracarboxylic acid and 1,2,3,4,5,6-cis-cyclohexylhexacarboxylic acid -based buffers.

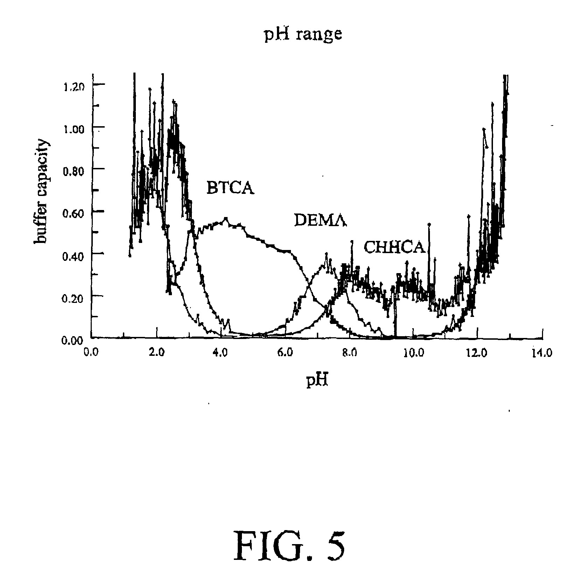

The buffer capacity curves were determined by titrating 0.4805 g H₂DEMA, 0.7026g H₄BTCA and 3.003 g H₆CHHCA, respectively, with 0.4242 M TMSOH, calculating the buffer capacity values and plotting them as a function of the pH as shown in Figure 5. It can be seen that only these three acids are needed to cover the entire pH range adequately.

All references cited herein are incorporated by reference. While this invention has been described fully and completely, it should be understood that, within the scope of the appended claims, the invention may be practiced otherwise than as specifically described. Although the invention has been disclosed with reference to its preferred embodiments, from reading this description those of skill in the art may appreciate changes and modification that may be made which do not depart from the scope and spirit of the invention as described above and claimed hereafter.

## Claims

1. A composition comprising a cationic salt of a weak acid, where the composition is metal atom or ion free and free of heteroatoms, except O or O and S and has volatile combustion products.

2. A composition as claimed in claim 1, wherein the weak acid is selected from the group consisting of carboxylic acids, phenols, half esters of sulfuric acid, and acidic hydroxy compounds and mixtures and combinations thereof, and the cationic salt is selected from the group consisting of oxonium ions, sulfonium ions, sulfoxonium ions and mixtures and combinations thereof.

3. A composition as claimed in claim 1 or 2, wherein the weak acid is selected from the group consisting of carboxylic acids and acidic hydroxy compounds and mixtures and combinations thereof, and the cationic salt is selected from the group consisting of sulfonium ions, sulfoxonium ions and mixtures and combinations thereof.

4. A composition as claimed in claim 2 or 3, wherein the carhoxylic acid is selected from the group consisting of mono-, oligo-, or polycarboxy alkanes, alkenes, or alkynes, mono-, oligo- or polycarboxy cycloalkanes, cycloalkene, cycloalkynes, mono-, oligo-, or polycarboxy aromatics, heteroatom-containing analogs thereof, where the heteroatoms are O and/or S, or mixtures or combinations thereof; wherein the sulfonium ions are selected from the group consisting of R,R',R"-sulfonium ions, where R, R' and R" are the same or different alkyl group having from 1 to about 30 carbon atoms, aryl group having from 6 to about 30 carbon atoms, aralkyl group having from 7 to about 30 carbon atoms, alkaryl group having from 7 to about 30 carbon atoms, a polyalkylene glycol group, a polyalkyleneoxide group, or mixtures or combinations thereof; and wherein the sulfoxonium ions are selected from the group consisting of R,R',R"-sulfoxonium ions, where R, R' and R" are the same or different alkyl group having from 1 to about 30 carbon atoms, aryl group having from 6 to about 30 carbon atoms, aralkyl group having from 7 to about 30 carbon atoms, alkaryl group having from 7 to about 30 carbon atoms, a polyalkylene glycol group, a polyalkyleneoxide group, or mixtures or combinations thereof.

5. A composition as claimed in claim 2 or 3, wherein the acidic hydroxy compounds include, without limitation, mono-, oligo-, or polyhydroxy alkanes, alkenes or alkynes, mono- or polyhydroxy cycloalkanes, cycloalkene, cycloalkynes, or aromatics and heteroatom-containing analogs, where the heteroatoms are O and/or S, or mixtures or combinations thereof; wherein the sulfonium ions are selected from the group consisting of R,R',R"-sulfonium ions, where R, R' and R" are the same or different alkyl group having from 1 to about 30 carbon atoms, aryl group having from 6 to about 30 carbon atoms, aralkyl group having from 7 to about 30 carbon atoms, alkaryl group having from 7 to about 30 carbon atoms, a polyalkylene glycol group, a polyalkyleneoxide group, or mixtures or combinations thereof; and wherein the sulfoxonium ions are selected from the group consisting of R,R',R"-sulfoxonium ions, where R. R' and R" are the same or different alkyl group having from 1 to about 30 carbon atoms, aryl group having from 6 to about 30 carbon atoms, aralkyl group having from 7 to about 30 carbon atoms, alkaryl group having from 7 to about 30 carbon atoms, a polyalkylene glycol group, a polyalkyleneoxide group, or mixtures or combinations thereof.

6. A composition as claimed in any one of the preceding claims, wherein the weak acid is selected from the group consisting of diethylmalonic acid, 1,2,3,4-butane tetracarboxylic acid, 3-hydroxy-2-methyl-4-pyrone, 4-hydroxy benzoic, carbonic acid, and cis-1,2,3,4,5,6-cyclohexyl hexacarboxylic acid; wherein the cation is selected from the group consisting of trimethylsulfonium ion, triethylsulfonium ion, tripropylsulfonium ion and tributylsulfonium ion.

7. A buffer composition for use in analytical systems having gas-phase or vapor-phase element-specific detectors (ESDs), where the composition comprises a compound comprising a cationic salt of a weak acid, where the composition is metal-atom-free and free of heteroatoms, except O and/or S and has volatile combustion products.

8. A composition as claimed in claim 7, comprising at least two compounds and covering a desired pH range between about 1 and about 13.

9. A composition as claimed in claim 7 or 8, wherein the weak acid is selected from the group consisting of carboxylic acids, phenols, half esters of sulfuric acid, and acidic hydroxy compounds and mixtures and combinations thereof, and the cationic salt is selected from the group consisting of oxonium ions, sulfonium ions, sulfoxonium ions and mixtures and combinations thereof.

10. A composition as claimed in any one of claims 7 to 9, wherein the weak acid is selected from the group consisting of carboxylic acids and acidic hydroxy compounds and mixtures and combinations thereof, and the cationie salt is selected from the group consisting of sulfonium ions, sulfoxonium ions and mixtures and combinations thereof.

11. A composition as claimed in claim 9 or 10, wherein the carboxylic acid is selected from the group consisting of mono-, oligo-, or polycarboxy alkanes, alkenes, or alkynes, mono-, oligo- or polycarboxy cycloalkanes, cycloalkene, cycloalkynes, mono-, oligo-, or polycarboxy aromatics, heteroatom-containing analogs thereof, where the heteroatoms are O and/or S, or mixtures or combinations thereof; wherein the sulfonium ions are selected from the group consisting of R,R',R"-sulfonium ions, where R, R' and R" are the same or different alkyl group having from 1 to about 30 carbon atoms, aryl group having from 6 to about 30 carbon atoms, aralkyl group having from 7 to about 30 carbon atoms, alkaryl group having from 7 to about 30 carbon atoms, a polyalkylene glycol group, a polyalkyleneoxide group, or mixtures or combinations thereof; and wherein the sulfoxonium ions ate selected from the group consisting of R,R',R"-sulfoxonium ions, where R, R' and R" are the same on different alkyl group having from 1 to about 30 carbon atoms, aryl group having from 6 to about 30 carbon atoms, aralkyl group having from 7 to about 30 carbon atoms, alkaryl group having from 7 to about 30 carbon atoms, a polyalkylene glycol group, a polyalkyleneoxide group, or mixtures or combinations thereof.

12. A composition as claimed in claim 9 or 10, wherein the acidic hydroxy compounds include, without limitation, mono-, oligo-, or polyhydroxy alkanes, alkenes or alkynes, mono- or polyhydroxy cycloalkanes, cycloalkene, cycloalkynes, or aromatics and heteroatom-containing analogs, where the heteroatoms are O and/or S, or mixtures or combinations thereof; wherein the sulfonium ions are selected from the group consisting of R,R',R"-sulfonium ions, where R, R' and R" are the same or different alkyl group having from 1 to about 30 carbon atoms, aryl group having from 6 to about 30 carbon atoms, aralkyl group having from 7 to about 30 carbon atoms, alkaryl group having from 7 to about 30 carbon atoms, a polyalkylene glycol group, a polyalkyleneoxide group, or mixtures or combinations thereof; and wherein the sulfoxonium ions are selected from the group consisting of R,R',R"-sulfoxonium ions, where R, R' and R" arc the same or different alkyl group having from 1 to about 30 carbon atoms, aryl group having from 6 to about 30 carbon atoms, aralkyl group having from 7 to about 30 carbon atoms, alkaryl group having from 7 to about 30 carbon atoms, a polyalkylene glycol group, a polyalkyleneoxide group, or mixtures or combinations thereof.

13. A composition as claimed in claim 9, wherein the weak acid is selected from the group consisting of diethylmalonic acid, 1,2,3,4-butane tetracarboxylic acid, 3-hydroxy-2-methyl-4-pyrone, 4-hydroxy benzoic, carbonic acid, and cis-1,2,3,4,5,6-cyclohexyl hexacarboxylic acid; wherein the cation is selected from the group consisting of trimethylsulfonium ion, triethylsulfonium ion, tripropylsulfonium ion and tributylsulfonium ion.

14. An analytical system for detecting an analyte containing heteroatom other than O and/or S comprising:
a combustion zone where an analyte and a buffer composition are converted to their corresponding volatile combustion products; and
a detector capable of detecting at least one of the corresponding volatile combustion products of the analyte,
where the buffer composition is as defined in any one of claims 7 to 13.

15. A system as claimed in claim 14, further comprising:
a analytical separation apparatus selected from the group consisting of a chromatographic separation apparatus, an electrophoretic separation apparatus, and an extractive separation apparatus or a flow-injection apparatus.

16. A system as claimed in claim 14 or 15, wherein the detector comprises an element-specific-detector.

17. A system as claimed in claim 16, wherein the element-specific detectors include nitrogen-selective gas-phase chemiluminescence detectors, sulfur-selective gas-phase chemiluminescence detectors, nitrogen-phosphorus thermoionic detectors, electron-capture detectors, atomic emission plasma detectors, or inductively-coupled plasma-mass spectrometric (ICP-MS) detectors.

18. A system as claimed in any one of claims 14 to 17, further comprising a transformation zone where at least one volatile combustion product of the sample is converted into ; transformate and a detector capable of detecting at least one transformate.

19. A method comprising the steps of combusting a sample and a buffer composition to their corresponding volatile combustion products in a combustion zone and detecting at least one sample volatile combustion products in a detector, where the buffer composition is as defined in any one of claims 7 to 13.

20. A method as claimed in claim 19, further comprising the step of mixing the sample and the buffer composition prior to combustion.

21. A method as claimed in claim 19 or 20, further comprising the step of converting at least one volatile combustion product of the sample into at least one transformate and detecting at least one of the transformates.
